(19) Europäisches Patentamt
European Patent Office
Office européen des brevets

(11) Publication number: **0 090 203**
**B1**

# (12) EUROPEAN PATENT SPECIFICATION

(45) Date of publication of patent specification: **12.11.86**

(51) Int. Cl.⁴: **C 07 D 317/58**

(21) Application number: **83102199.3**

(22) Date of filing: **06.03.83**

## (54) Process for preparing p.chlorophenoxyacetyl-piperonylpiperazine.

(30) Priority: **29.03.82 IT 2044282**

(43) Date of publication of application:
**05.10.83 Bulletin 83/40**

(45) Publication of the grant of the patent:
**12.11.86 Bulletin 86/46**

(84) Designated Contracting States:
**AT BE CH DE FR GB LI LU NL SE**

(56) References cited:
**FR-A-1 311 316**
**FR-M- 1 538**
**FR-M- 7 524**

(73) Proprietor: **RAVIZZA S.p.A.**
**35, Via Europa**
**I-20053 Muggio' (Milano) (IT)**

(72) Inventor: **Mauri, Francesco**
**VIa Boito, 65**
**Monza (Milan) (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 090 203 B1

**Description**

This invention relates to an improved process for preparing the compound p.chlorophenoxyacetyl-piperonylpiperazine ("Fipexide") of formula:

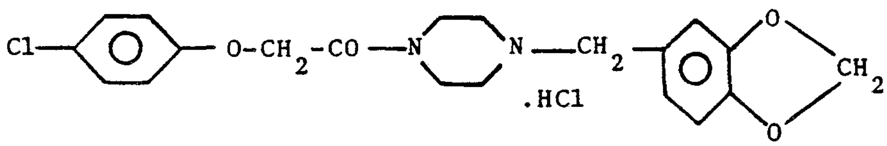

This compound is known for its therapeutic properties based on its antidepressive action and stimulating action on the nervous system, and in comparison with other compounds of the same class and similar therapeutic effect is distinguished by its very low toxicity both when administered orally and subcutaneously. Details of the therapeutic use of this compound can be obtained from French medicament patent No. 7524 M.

The process according to the present invention consists of reacting p.chlorophenoxyacetyl chloride with piperonylpiperazine in a solvent which is inert towards the reagents, such as toluene, benzene, cyclohexane or chloroform.

The reaction is as follows:

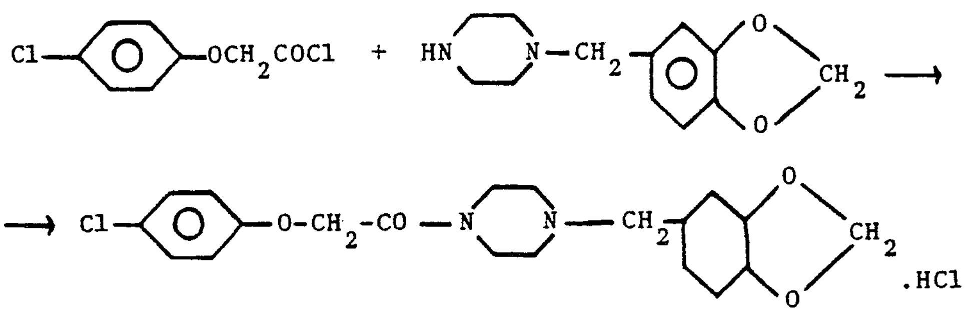

The use of chloroform as the reaction solvent has proved particularly useful. This solvent enables the product in crystalline form to be separated from the reaction medium very easily and reliably.

In contrast to what an expert of the organic synthesis art might expect, it has been found that in said reaction it is not necessary to use a basic substance acting as an acceptor for the hydrochloric acid formed in the reaction.

Instead, it has been surprisingly found that the process according to the invention gives clearly better results in terms of yield than the results indicated in the aforesaid French patent, which operates in the presence of pyridine as the HCl fixing base.

In practice, yields can be obtained with the new process which are close to 100% in comparison with the approximately 60% yield according to the French patent.

Dispensing with the use of pyridine (or with another basic substance) not only represents a saving of reagents, but also prevents the formation of a non-volatile by-product such as pyridine hydrochloride, which requires costly operations for its separation from the required reaction product.

In this respect, in the known art the pyridine hydrochloride is eliminated by evaporating the reaction solvent, re-dissolving the residue in $CHCl_3$, then washing the solution obtained in this manner with an aqueous solution of $Na_2CO_3$, then with water and finally with a 10% hydrochloric acid solution.

In the process according to the present invention, the reagents can be used in stoichiometric proportions. A small excess of p.chlorophenoxyacetyl chloride is however advantageous.

The p.chlorophenoxyacetyl chloride used as the reagent in said reaction can itself be prepared by chlorinating p.chlorophenoxyacetic acid with thionyl chloride in the presence of a small quantity of pyridine of the order of 1.5% of the acid by weight, and in the presence of a solvent which can advantageously be chloroform.

The reaction mixture is heated to boiling, to thus remove the gaseous by-products $SO_2$ and HCl. The acid chloride is obtained in the form of a solution in the solvent used, and can be utilised as such in the process according to the invention.

According to a preferred alternative, the p.chlorophenoxyacetyl chloride is prepared directly in the reaction mixture comprising the piperonylpiperazine, from p.chlorophenoxyacetic acid and thionyl chloride.

The following reaction is carried out in practice:

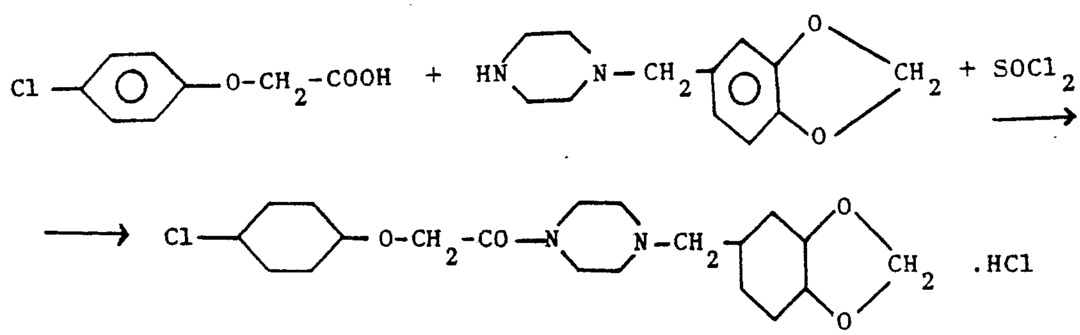

The ability to carry out the process in a single stage by this alternative method, which is extremely advantageous, could not be foreseen because the formation of the acid chloride gives rise to HCl, which salifies the amine and thus theoretically reduces or nullifies its reactivity.

The following example is given as a purely illustrative embodiment of the invention.

Example 1

Preparation of p.chlorophenoxyacetyl chloride:

165 kg of chloroform, 33 kg (177 moles) of p.chlorophenoxyacetic acid and 0.5 kg (6.33 moles) of pyridine are fed into a 250 l enamelled reactor, the mixture is heated to boiling, and 22.7 kg (190 moles) of thionyl chloride are then added at a rate of 2 l/h.

When the $SOCl_2$ has been added, the temperature is maintained at boiling point until the acid vapours ($SO_2$, HCl) have been eliminated.

A solution of the acid chloride in chloroform is obtained in this manner, and can be used directly in preparing the "Fipexide".

Preparation of Fipexide:

36 kg (164 moles) of piperonylpiperazine dissolved in 100 kg of chloroform are fed into a 1000 l enamelled reactor. The solution of the acid chloride in chloroform, which was obtained in the preceding example and consisting of 36 kg (175 moles) of the acid chloride dissolved in 132 kg of chloroform, is then added over a period of 30 minutes. The mixture temperature is allowed to rise to boiling point and is maintained at that temperature for 2 hours.

It is then cooled, the dense mass is diluted with 100 kg of chloroform, 120 l of water are added and the mixture is centrifuged. The separated crystalline product is then recrystallised from 200 l of 70% ethanol. The product is centrifuged and washed with 100 l of water. It is dried to give approximately 60—62 kg of product. A further 5—6 kg of product are recovered by evaporating the liquors. A total of approximately 65 kg of product is obtained with a yield of approximately 93%. The mother liquors of the first centrifuging stage are alkalised, the chloroform separated and recovered by distillation.

Example 2

Preparation of Fipexide.

35 kg (159 moles) of piperonylpiperazine, 29.7 kg (159 moles) of p.chlorophenoxyacetic acid and 495 kg of chloroform are fed into a 1000 l enamelled reactor. The mixture is heated to boiling, and 22.7 kg of thionyl chloride are poured in.

The mixture is stirred under reflux until hydrochloric acid ceases to be evolved. The mixture is cooled, 110 l of water are added, and the mixture centrifuged.

The product obtained is crystallised from 300 l of 80% ethanol. 60 kg of Fipexide hydrochloride are obtained, equivalent to a yield of 88%.

**Claims**

1. A process for preparing p.chlorophenoxyacetyl-piperonylpiperazine, by reacting p.chlorophenoxyacetyl chloride directly with piperonylpiperazine in the presence of an inert solvent, characterized by the absence of substances acting as acid acceptors, and raising the temperature of the reaction mixture to boiling point.

2. A process as claimed in claim 1, characterized by using chloroform as the reaction solvent.

3. A process as claimed in claim 2, characterized by using the p.chlorophenoxyacetyl chloride in the form of a solution in chloroform, the solution being obtained directly by chlorinating p.chlorophenoxyacetic acid with thionyl chloride in the presence of chloroform.

4. A process as claimed in claim 1, characterized by forming the p.chlorophenoxyacetyl chloride in the reaction mixture, from p.chlorophenoxyacetic acid and thionyl chloride.

## Patentansprüche

1. Verfahren zum Herstellen von p.Chlorphenoxyacetylpiperonylpiperazin durch Umsetzen von p.Chlorphenoxyacetylchlorid direkt mit Piperonylpiperazin in Anwesenheit eines inerten Lösungsmittels, gekennzeichnet durch das Fehlen von Substanzen, die als Säureakzeptoren wirken und durch Erhöhen der Temperatur der Reaktionsmischung auf den Siedepunkt.

2. Verfahren, wie in Anspruch 1 beansprucht, gekennzeichnet durch die Verwendung von Chloroform als Reaktionslösungsmittel.

3. Verfahren, wie in Anspruch 2 beansprucht, gekennzeichnet durch die Verwendung des p.Chlorphenoxyacetylchlorids in Form einer Lösung in Chloroform, wobei die Lösung direkt durch Chlorieren von p.Chlorphenoxyessigsäure mit Thionylchlorid in Anwesenheit von Chloroform erhalten wird.

4. Verfahren, wie in Anspruch 1 beansprucht, gekennzeichnet durch Bildung des p.Chlorphenoxyacetylchlorids in der Reaktionsmischung aus p.Chlorphenoxyessigsäure und Thionylchlorid.

## Revendications

1. Procédé de préparation de la p-chlorophénoxyacétylpipéronylpipérazine, par réaction du chlorure de p-chlorophénoxyacétyle directement avec la pipéronylpipérazine en présence d'un solvant inerte, caractérisé par l'absence de substances agissant comme accepteurs d'acide, et en ce qu'on élève la température du mélange réactionnel au point d'ébullition.

2. Procédé selon la revendication 1, caractérisé en ce qu'on utilise du chloroforme comme solvant de réaction.

3. Procédé selon la revendication 2, caractérisé en ce qu'on utilise du chlorure de p-chlorophénoxyacétyle sous forme d'une solution dans le chloroforme, la solution étant obtenu directement par chloration de l'acide p-chlorophénoxyacétique par le chlorure du thionyle en présence de chloroforme.

4. Procédé selon la revendication 1, caractérisé en ce qu'on forme le chlorure de p-chlorophénoxyacétyle dans le mélange réactionnel, à partir de l'acide p-chlorophénoxyacétique et du chlorure de thionyle.